# EUROPEAN PATENT APPLICATION

(11) **EP 3 187 108 A1**
(43) Date of publication of application: **05.07.2017**
(21) Application number: 15836196.4
(22) Date of filing: 20.08.2015
(51) Int. Cl.: A61B 5/0245, G04G 99/00

(54) **BIOLOGICAL INFORMATION MEASURING DEVICE**

(30) Priority: 27.08.2014 JP 2014173296
(71) Applicant: Seiko Epson Corporation, Tokyo 160-8801 (JP)
(72) Inventor: ICHIKAWA, Masahito, Suwa-shi Nagano 392-8502 (JP)
(74) Representative: Hoffmann Eitle
(86) International application number: PCT/JP2015/004154
(87) International publication number: WO 2016/031193

(57) **Abstract**

Restraining of power consumption and reduction in load are to be achieved by causing a pulse wave sensor to a minimum necessary extent and not performing unnecessary processing, in response to each user measuring the heart rate for various purposes.

A biological information measuring device 1 includes: a pulse wave sensor unit 160 which detects a pulse wave of a living body and outputs a pulse wave signal; an MCU 136 which generates heart rate information and time information; a storage unit 159 capable of storing the heart rate information; operation buttons 151 to 154 which output an operation signal corresponding to an input operation by a person; and a display unit 120. The MCU 136 displays the time information on the display unit 120 in a time display mode, writes the heart rate information in the storage unit 159 in a measuring mode, and displays the heart rate information on the display unit 120 without writing the heart rate information in the storage unit 159 in a heartbeat display mode. The MCU 136 starts processing to shift the biological information measuring device from the time display mode to the heartbeat display mode if the operation signal indicates a predetermined operation.

## Description

### Technical Field

The present invention relates to a biological information measuring device.

### Background Art

Recently, a wristwatch-type measuring device capable of measuring the heart rate of the user has been widely used. Such a measuring device has a pulse wave sensor, for example, and detects the pulse wave of the user with this pulse wave sensor. For example, PTL 1 discloses a measuring device having a pulse wave sensor fixed to the user's finger. Since the heart rate can be measured on the basis of the pulse wave, the user can check the heart rate and grasp his/her physical condition to a certain extent by wearing this measuring device.

### Citation List

### Patent Literature

PTL 1: JP-A-2003-265441

### Summary of Invention

### Technical Problem

Incidentally, the function of measuring the heart rate in the traditional measuring device is aimed at analyzing and recording the heart rate during a specific time period, for example, I during exercise.

Meanwhile, many users consider that it is enough if they can check their heart rate at a certain time point in order to grasp their physical condition in everyday life. For these users, analysis and recording of measurement results are not necessary and it is enough as long as their heart rate is displayed when they want to check it.

Also, in the traditional measuring device, during the period when the device is set in a mode for measuring the heart rate, the pulse wave sensor is constantly in operation and the analysis and recording of measuring results are carried out as well. Therefore, the power consumed by the measuring device increases and the memory is used excessively.

Since the biological information measuring device is used in the state of being worn on the body, a convenient configuration which achieves a smaller size, smaller thickness, and longer duration of power supply than the traditional device is desired. Specifically, it is desired that the pulse wave sensor is housed in an outer case to reduce the size and thickness and that the duration of power supply is secured as well.

In view of the foregoing circumstances, a problem to be solved by the invention is to achieve at least one of portability (smaller size and smaller thickness), visibility of display, and durability of power, thus improving convenience.

### Solution to Problem

In order to solve the foregoing problem, a biological information measuring device according to an aspect of the invention includes: a pulse wave sensor unit which detects a pulse wave of a living body and outputs a pulse wave signal; a heart rate information generation unit which generates heart rate information indicating a heart rate on the basis of the pulse wave signal; a time information generation unit which generates time information indicating time; a storage unit capable of storing the heart rate information; an operation unit which outputs an operation signal corresponding to an input operation by a person; a display unit; and a control unit which displays the time information on the display unit in a first mode, writes the heart rate information in the storage unit in a second mode, and displays the heart rate information on the display unit without writing the heart rate information in the storage unit in a third mode. The control unit starts processing to shift the biological information measuring device from the first mode to the third mode if the operation signal indicates a predetermined operation.

According to this aspect, when the user temporarily wants to know the heart rate at the time point, the user shifts the biological information measuring device to the third mode instead of the second mode. Thus, compared with when the biological information measuring device is shifted to the second mode, excessive use of the storage capacity of the storage unit is prevented and the processing load involved in the processing to write the heart rate information in the storage unit is saved.

A biological information measuring device according to another aspect of the invention is the biological information measuring device according to the one aspect, in which the control unit sets the pulse wave sensor unit in a halt state in the first mode, and sets the pulse wave sensor unit in an operating state in the second mode and the third mode.

According to this aspect, since the pulse wave sensor unit is set in the halt state in the first mode, if the biological information measuring device is shifted to the first mode when the heart rate information is not needed, the power consumption and processing load required when setting the pulse wave sensor unit in the operating state are saved.

A biological information measuring device according to another aspect of the invention is the biological information measuring device according to the one aspect, which includes a receiving unit for receiving position information from a satellite and in which the control unit sets the receiving unit in an operating state in the second mode and sets the receiving unit in a halt state in the third mode.

According to this aspect, the receiving unit is set in the halt state in the third mode. Therefore, when the user does not need information such as traveling distance or travelling speed and temporarily wants to know the heart rate at the time point, the user shifts the biological information measuring device to the third mode instead of the second mode. Thus, compared with when the biological information measuring device is shifted to the second mode, the power consumption and processing load required when setting the receiving unit in the operating state are saved.

A biological information measuring device according to another aspect of the invention is the biological information measuring device according to the one aspect, in which the operation unit has a plurality of buttons including a first button and in which the predetermined operation is an operation on the first button.

According to this aspect, a configuration in which when an operation on the first button is carried out, the biological information measuring device shifts from the first mode to the third mode, that is, a configuration in which the biological information measuring device shifts to the third mode by a simple operation, is realized.

A biological information measuring device according to another aspect of the invention is the biological information measuring device according to the one aspect, in which the plurality of buttons includes a second button and in which the control unit shifts the biological information measuring device from the first mode to the second mode if the operation signal indicates an operation on the second button.

According to this aspect, a configuration in which when an operation on the second button is carried out, the biological information measuring device shifts from the first mode to the second mode, that is, a configuration in which the biological information measuring device shifts to the second mode by a simple operation, is realized. Also, since the button (second button) having the function of shifting to the second mode and the button (first button) having the function of shifting the third mode are provided separately, an operation error is prevented.

A biological information measuring device according to another aspect of the invention is the biological information measuring device according to the one aspect, in which the control unit shifts the biological information measuring device from the third mode to the first mode when a predetermined time has passed after the shift to the third mode.

According to this aspect, even if an operation to return (shift) the biological information measuring device to the first mode is not carried out after the biological information measuring device shifts to the third mode, the biological information measuring device is forcedly shifted to the first mode when a predetermined time has passed. Therefore, the pulse wave sensor unit is prevented from unnecessarily remaining set in the operating state.

A biological information measuring device according to another aspect of the invention is the biological information measuring device according to the one aspect, in which the control unit sets the predetermined time on the basis of the operation signal.

According to this aspect, the user can set the predetermined time until the biological information measuring device shifted to the third mode is forcedly shifted to the first mode by so-called time-out processing, to a time period intended by the user. Therefore, the pulse wave sensor unit is prevented from being set in the halt state against the user's intention.

A biological information measuring device according to another aspect of the invention is the biological information measuring device according to the one aspect, in which the control unit shifts the biological information measuring device from the third mode to the first mode if the operation signal indicates an operation on the first button after the shift to the third mode.

According to this aspect, a configuration in which when an operation on the first button is carried out, the biological information measuring device shifts from the first mode to the third mode, that is, a configuration in which the biological information measuring device shifts to the third mode by a simple operation, is realized.

A biological information measuring device according to another aspect of the invention is the biological information measuring device according to the one aspect, which includes a determination unit which determines whether the biological information measuring device is worn on the living body or not, on the basis of the pulse wave signal, and in which the control unit maintains the first mode if it is determined by the determination unit that the biological information measuring device is not worn on the living body, even when processing to shift from the first mode to the third mode is started.

According to this aspect, if the biological information measuring device is not worn on the living body, even when the processing to shift from the first mode to the third mode is started, the control unit does not shift the biological information measuring device to the third mode. Thus, an excessive increase in power consumption due to the setting of the pulse wave sensor unit in the operating state despite the biological information measuring device not being worn on the living body is prevented.

### Brief Description of Drawings

[FIG. 1] FIG. 1 is a cross-sectional view of a biological information measuring device according to an embodiment of the invention.
[FIG. 2] FIG. 2 is a perspective view of the biological information measuring device according to the embodiment of the invention.
[FIG. 3] FIG. 3 is a plan view of the biological information measuring device according to the embodiment of the invention.
[FIG. 4] FIG. 4 is a view showing the system configuration of the biological information measuring device according to the embodiment of the invention.
[FIG. 5] FIG. 5 is a view showing a display example on a display unit when the biological information measuring device is in a "time display mode".
[FIG. 6] FIG. 6 is a view showing a display example on the display unit when the biological information measuring device is in a "measuring mode".
[FIG. 7] FIG. 7 is a view showing a display example on the display unit when the biological information measuring device is in a "heartbeat display mode", and a display example of a "preparation screen".
[FIG. 8] FIG. 8 is a view showing a flowchart of processing for switching operation modes.
[FIG. 9] FIG. 9 is a view showing a flowchart of processing to shift to heartbeat display mode (subroutine of Step S102) .
[FIG. 10] FIG. 10 is a view showing an example of a screen displayed on the display unit when the user sets a zone.
[FIG. 11] FIG. 11 is a view showing an example of a screen displayed on the display unit when the user sets a zone.
[FIG. 12] FIG. 12 is a view for explaining a setting example of numerical ranges of zones.
[FIG. 13] FIG. 13 is a view showing a display example on the display unit in the measuring mode in the case where numerical ranges of zones are set in an overlapping manner.
[FIG. 14] FIG. 14 is a view showing a display example on the display unit when a heart rate alarm is actuated.

### Description of Embodiments

Hereinafter, an embodiment of the invention will be described. The embodiment described below should not unduly limit the contents of the invention described in the claims. Not all the configurations described in the embodiment are necessarily essential components of the invention, either.

### 1. Technique in This Embodiment

First, the technique in this embodiment will be described. A technique in which biological information is acquired using a photoelectric sensor in a wearable biological information measuring device (so-called running watch) worn by the user on the wrist or the like is known. As a biological sensor which is a photoelectric sensor, a pulse wave sensor is conceivable, for example. By using the pulse wave sensor, it is possible to acquire a pulse wave signal such as the pulse rate.

Hereinafter, a description is given using a wristwatch-type device worn on the wrist as an example. However, the biological information measuring device according to the embodiment may also be worn by the user on other parts such as the neck or ankle. Also, the biological sensor (photoelectric sensor) in the embodiment is not limited to a pulse wave sensor, and a photoelectric sensor which acquires biological information other than a pulse wave signal may be used. Also, the biological information measuring device in the embodiment may include a biological sensor other than a photoelectric sensor.

In the biological information measuring device including the photoelectric sensor, it is necessary to receive necessary light and block unnecessary light. In the example of the pulse wave sensor, reflected light reflected by a subject (particularly a part containing a measurement target blood vessel) includes a pulse wave component and therefore should be received, but the other light is noise component and therefore should be blocked. Here, as the "other light", direct light which is emitted from a light emitting unit and becomes directly incident on a light receiving unit, reflected light reflected by objects other than the subject, or ambient light such as sunlight or illumination light is conceivable.

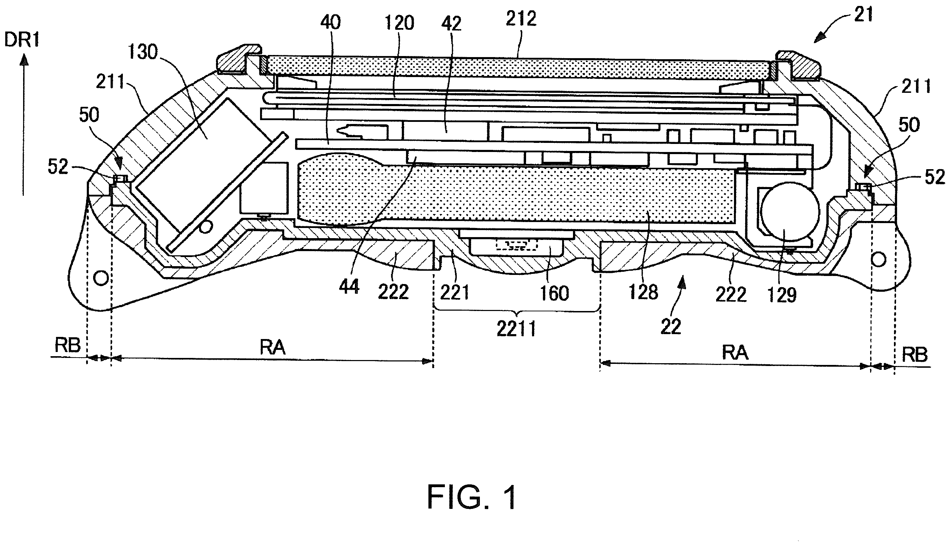

In order to properly control such light transmission and light blocking, the biological information measuring device may include a light transmitting part and a light blocking part. As an example, the arrangement or the like of a light transmitting part and a light blocking part in a section (in a narrow sense, the bottom case) provided on the side of the subject of the biological information measuring device, as described later using FIG. 1 and the like, may be taken into account.

However, when the use case of the biological information measuring device according to the embodiment is considered, satisfying various other requirements than the positional relation between the light transmitting part and the light blocking part enables implementation of an appropriate device.

First, high waterproofness is required in the biological information measuring device. Inside the biological information measuring device (in the example of FIG. 1, the space between a top case 21 and a bottom case 22), a circuit board 40, a battery (secondary battery 128), a vibrating motor (vibrating part 129) and the like are included. Therefore, if waterproofness is low, there is a risk of failure in these parts. Particularly a wearable device such as wristwatch type may be considered to be worn at the time exercise and used to present information such as exercise intensity. In such a case, the skin surface of the user is often wet with sweat, and the risk of inflow of a liquid or gas such as water vapor into the device should be restrained.

On one side of the circuit board 40, a panel frame 42 for guiding a display panel such as a display unit 120 is arranged, and a circuit case 44 for guiding the secondary battery 128 or the like is arranged on the other side.

For the circuit board 40, a glass fiber-containing epoxy resin-based substrate or the like is used, and wiring patterns made of copper foils are formed on both sides. Meanwhile, for the panel frame 42 and the circuit case 44, a resin such as polyacetal or polycarbonate is used.

On the circuit board 40, elements forming a circuit for driving the photoelectric sensor to measure the pulse rate, a circuit for driving the display unit 120, and a circuit for controlling each circuit are mounted. On one side of the circuit board 40, an electrode for connection to the display unit 120 is formed and is electrically continuous to an electrode of the display unit 120 via a connector, not shown.

On the display unit 120, pulse rate measurement data such as pulse rate, and time information such as current time, or the like is displayed according to each operation mode. Each operation mode will be described later.

In the circuit case 44, a rechargeable button-type secondary battery 128 (lithium secondary battery) is stored. The secondary battery 128 has both pole terminals connected to the circuit board 40 and supplies power to a circuit for controlling power supply. The power is converted to a predetermined voltage or the like in this circuit and then supplied to each circuit, thus actuating the circuit for driving the photoelectric sensor to detect the pulse rate, the circuit for driving the display unit 120, the circuit for controlling each circuit, and the like. The recharging of the secondary battery 128 is carried out via a pair of recharging terminals that is made electrically continuous to the circuit board 40 by a continuation member such as a coil spring. Although an exampling using the secondary battery 128 as a battery is described here, a primary battery that needs no recharging may be used as a battery.

It is assumed that measurement of exercise state and measurement of the degree of healthiness, for example, are included in the application of the biological information measuring device 1 according to the embodiment. Therefore, the applicant realizes the biological information measuring device 1 that can be continuously used for a long time by considering the configurations of and control methods for a pulse wave sensor unit 160, the circuit board 40 or other components. As the secondary battery 60 described later, a 150-mAh secondary battery can be used, for example.

Second, the strength of the case parts (top case and bottom case) needs to be high. While various components are arranged inside the device as described above, various forces are applied to the wearable biological information measuring device, linked with movements of the user. For example, if the user us jogging or the like, a pressing force or twisting force may be applied to the device, due to an arm swinging movement. In this case, if this force is applied to internal components such as the circuit board 40, it may lead to the failure of the component.

Third, wearing comfort for the user needs to be good. The wearable biological information measuring device needs to be worn by the user at the time of use. If the biological information measuring device is used at the time of exercise as described above, the wearing needs to continue during the period for which data acquisition is desired (for example, from the start to the end of exercise). Alternatively, in an example where the degree of healthiness of the user is to be determined, biological information needs to be acquired continuously for a long time (for example, a time span such as 12 hours, 24 hours or several days), and the device is worn during this period. Therefore, it is not preferable that the wearing of the biological information measuring device obstructs the user's exercise or everyday life, and good wearing comfort is an important factor. Specifically, the biological information measuring device should have a small sized (thin) and light weight. That is, it is desirable that the biological information measuring device can receive necessary light and block unnecessary light, has high waterproofness and strength, and has a small size and light weight.

In FIG. 1, the case part 20 is formed by the top case 21 and the bottom case 22, and the bottom case 22 has a structure with a light transmitting part 221 and a light blocking part 222. However, the case part is not limited to this. For example, the case part 20 may be formed by a single unified member. Also, various modifications can be made, such as forming the case part 20 with a top plate, which is a transparent plate-like member, and a resin member combined with the top plate. Hereinafter, in this description, it is assumed that the case part 20 has the structure of FIG. 1. However, the technique in the embodiment can also applied to the case where the case part 20 having another structure with the light transmitting part 221 and the light blocking part 222 is used. Also, in FIG. 1, the configurations of the pulse wave sensor unit 160 and a detection window 2211 and around the detection window 2211 are simplified.

In such a case, with the light transmitting part 221 and the light blocking part 222, the photoelectric sensor can use proper light for measurement. In this case, if the light transmitting part 221 and the light blocking part 222 are formed in a unified manner, the preparation of the case part 20 (particularly the bottom case 22) becomes easier.

In terms of the above first and second requirements, it suffices that the case part 20 is less deformable. If the case part 20 can easily be deformed, a path for liquid or water vapor to flow in may be generated by the deformation, or an external pressure can easily be transmitted to internal components. As the inflow path in this case, the gap between the light transmitting part 221 and the light blocking part 222 is conceivable. For example, even if the light transmitting part 221 and the light blocking part 222 are formed by two-color molding using a common resin base, these parts are bonded to each other with their surfaces slightly melted, and therefore water vapor or the like can enter the small gap between these parts. However, if the case part 20 is less deformable, these circumstances can be restrained. Generally, a less deformable structure can be achieved by increasing the thickness of the members. However, that makes is difficult to realize a small size and light weight of the biological information measuring device, and the above third requirement cannot be dealt with.

In this respect, in the embodiment, since the light blocking part 222 is made of a glass-containing resin material, the light blocking part 222 can be made less deformable. Therefore, there is no need to increase the thickness of the light blocking part 222, and a reduction in the thickness and weight of the biological information measuring device 1 itself can be realized while high waterproofness and strength are achieved. That is, since the light blocking part 222 is made of a glass-containing resin material, it is possible to efficiently solve the various problems described above.

Moreover, as the biological information measuring device 1 is reduced in thickness, the effect that the detection accuracy for biological information is increased (reduction in accuracy is restrained) can be expected. This is because, if the biological information measuring device 1 is thick, during the season when the user wears long-sleeved clothes, the device touches the sleeve and the device itself swings according to the movement of the sleeve. While the biological sensor such as the pulse wave sensor should be used in tight contact with the skin, if the device swings, the device may float up, thus lowering its measurement accuracy. In this respect, if the biological information measuring device 1 is reduced in thickness, the floating of the device due to the contact with the sleeve or the like can be restrained and detection accuracy can be increased.

Hereinafter, a specific example of the configuration of the biological information measuring device 1 according to the one embodiment will be described.

### 2. Configuration of Biological Information Measuring Device

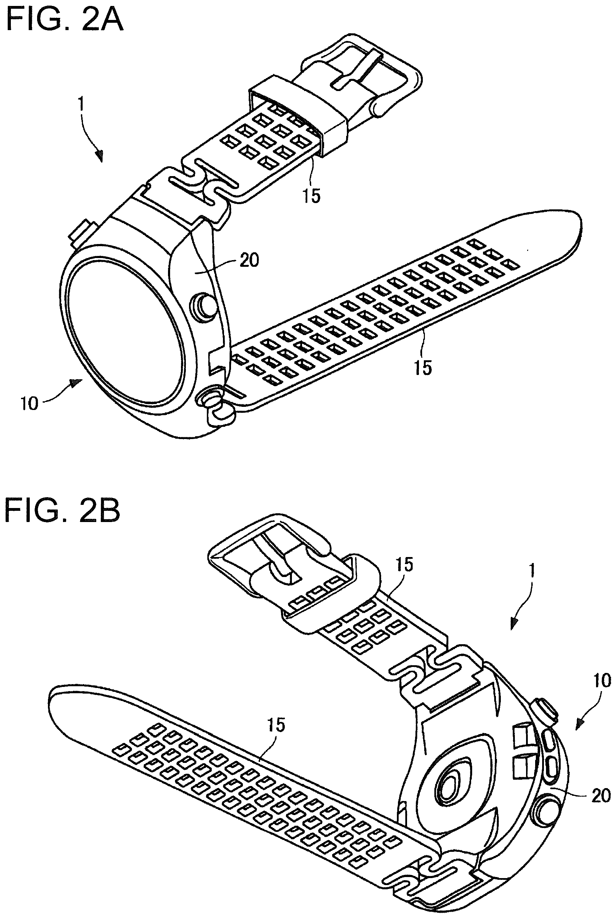

FIG. 2(A) and FIG. 2(B) show perspective views of the biological information measuring device 1 according to the embodiment. FIG. 2 (A) is a perspective view as seen from the side of the top case 21. FIG. 2(B) is a perspective view as seen from the side of the bottom case 22. The biological information measuring device 1 according to the embodiment is worn by the user on a predetermined part (for example, the wrist) and detects biological information such as pulse wave signal. The biological information measuring device 1 has a device main body 10 which comes in tight contact with the user and detects the biological information, and a band part 15 which is attached to the device main body 10 and configured to allow the device main body 10 to be worn by the user.

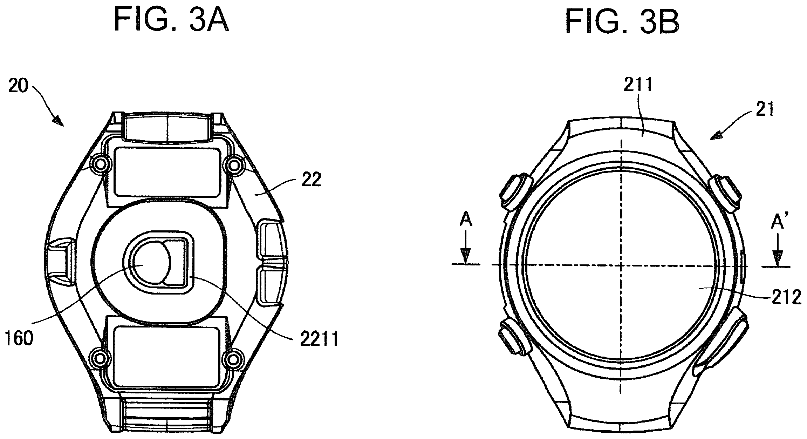

The device main body 10 has the top case 21 and the bottom case 22. FIG. 3(A) and FIG. 3(B) are views showing the part of the device main body 10 of the biological information measuring device 1. FIG. 3 (A) is a plan view as viewed in the direction from the bottom case 22 toward the top case 21, that is, in the direction of observing the biological information measuring device 1 from the side of the subject (user's wrist) in the state where the biological information measuring device 1 is worn by the user and thus used. FIG. 3 (B) is a plan view as viewed from the side opposite to FIG. 3 (A), that is, in the direction from the top case 21 toward the bottom case 22. That is, FIG. 3(A) is a plan view mainly showing the structure of the bottom case 22, and FIG. 3 (B) is a plan view mainly showing the structure of the top case 21.

As shown in FIG. 3(A), the detection window 2211 is provided in the bottom case 22, and the pulse wave sensor unit 160 is provided at a position corresponding to the detection window 2211. The detection window 2211 is configured to transmit light. The light emitted from a light emitting unit of a photoelectric sensor (sensor for detecting a pulse wave) provided in the pulse wave sensor unit 160 is transmitted through the detection window 2211 and cast onto the subject. Also, the light reflected by the subject is transmitted through the detection window 2211 as well and received by a light receiving unit of the pulse wave sensor. That is, the provision of the detection window 2211 enables detection of biological information using the photoelectric sensor. Specifically, the detection window 2211 may be realized by the light transmitting part 221 (see FIG. 1) (the light transmitting part 221 may include the detection window 2211). The specific structure of the light transmitting part 221 will be described later.

As show in FIG. 3 (B), the top case 21 may include a trunk part 211 and a glass plate 212. In this case, the trunk part 211 and the glass plate 212 may be used as an outer wall to protect the internal structure and may be configured to enable the user to view the display on a liquid crystal display (the display unit 120 shown in FIG. 1) provided immediately below the glass plate 212 through the glass plate 212. That is, in the biological information measuring device 1 of the embodiment, various kinds of information such as detected biological information or information indicating the exercise state, or time information, may be displayed using the display unit 120, and this display may be presented to the user from the side of the top case 21. While an example of realizing the top plate part of the biological information measuring device 1 with the use of the glass plate 212 is given here, the top plate part can be formed of materials other than glass, such as transparent plastic, as long as these materials are transparent members enabling the viewing of the display unit 120 and having a high enough strength to be able to protect the configuration included inside the case part 20 such as the display unit 120.

Next, an example of the detailed cross-sectional structure of the device main body 10 (see FIG. 2) of the biological information measuring device 1 will be described, using FIG. 1. FIG. 1 is a cross-sectional view taken along A-A' in FIG. 3(B). The top side on the face of FIG. 1 is the side of the top case 21, and the bottom side on the face of FIG. 1 is the side of the bottom case 22.

As shown in FIG. 1, the device main body 10 includes the pulse wave sensor unit 160, the circuit board 40, the panel frame 42, the circuit case 44, the secondary battery 128, the display unit 120, the vibrating part 129, and an antenna 130, in addition to the top case 21 and the bottom case 22. However, the configuration of the biological information measuring device 1 is not limited to FIG. 1. Other configurations can be added and a part of the configuration may be omitted.

The pulse wave sensor unit 160 has a photoelectric sensor, as described above. Thus, in the biological information measuring device 1, since the pulse wave sensor unit 160 has a photoelectric sensor, due to its properties, a pulse wave can be measured as biological information, for example, and on the basis of this, the pulse rate, stiffness of blood vessels, exercise-related state, mental state and the like can be derived.

The photoelectric sensor condenses, with a condensing mirror, the light cast toward the user's wrist from a light emitting unit such as an LED (light emitting diode) and reflected by a blood vessel in the wrist, and receives the light with a light receiving unit such as a photodiode. At this time, the photoelectric sensor measures the pulse rate of the user, utilizing a phenomenon of light reflectance varying between the expansion and contraction of the blood vessel. Based on this, it is preferable that the pulse wave sensor unit 160 is pressed against the wrist, and more preferably, in tight contact with the wrist, so that the light forming a measurement noise will not be received by the light receiving element of the photoelectric sensor.

On one side of the circuit board 40, the panel frame 42 for guiding the display panel such as the display unit 120 is arranged, and the circuit case 44 for guiding the secondary battery 128 or the like is arranged on the other side.

For the circuit board 40, a glass fiber-containing epoxy resin-based substrate or the like is used, and wiring patterns made of copper foils are formed on both sides. Meanwhile, for the panel frame 42 and the circuit case 44, a resin such as polyacetal or polycarbonate is used.

On the circuit board 40, an MCU (memory control unit) 136 made up of various ICs for controlling the display on the display unit 120 and processing satellite signals received by the antenna 30 is mounted. Under the control of a main control installed in the MCU 136, traveling information such as traveling speed, traveling distance, traveling duration, traveling pace (for example, time (minutes) required per km), pitch (number of steps taken per minute), and number of steps taken, is displayed on the display unit 120. An electrode for connection to the display unit 120 is formed on one side of the circuit board 40 and is electrically continuous to an electrode of the display unit 120 via a connector, not shown.

As described above, the biological information measuring device 1 according to the embodiment includes the secondary battery 128 provided in the case part 20, and the circuit board 40 which is provided on the side opposite to the subject contact surface side of the secondary battery 128, in the case part 20, and on which the processing device of the biological information measuring device 1 is mounted. If the biological information measuring device 1 has the configuration of FIG. 1, it can also be said that the biological information measuring device 1 includes the secondary battery 128 provided between the top case 21 and the bottom case 22, and the circuit board 40 which is provided between the secondary battery 128 and the top case 21 and on which the processing device of the biological information measuring device 1 is mounted. Here, the secondary battery 128 and the circuit board 40 may be provided in a center part of the biological information measuring device 1, as viewed in a plan view seen from the subj ect contact surface side (corresponding to FIG. 3(A)).

Also, the biological information measuring device 1 may include the vibrating part 129 (vibrating motor) provided more closely to an end of the biological information measuring device 1 than the secondary battery 128, as viewed in a plan view of the case part 20 (in a narrow sense, the bottom case 22) seen from the subject contact surface side. The vibrating part 129 may notify the user in a certain way, for example, and can be used as a user interface that is different from the display unit 120. In the example of FIG. 1, the vibrating part 129 is provided more closely to the right end than the secondary battery 128.

Next, details of the cross-sectional structure of the light transmitting part 221 and the light blocking part 222 will be described. As can be seen from FIG. 1, the light blocking part 222 is provided in such a way as to cover the light transmitting part 221 from the subject side, in the part excluding the detection window 2211.

In the detection window 2211, the light transmitting part 221 is not covered by the light blocking part 222. In other words, the detection window 2211 is realized by the light transmitting part 221. Therefore, as described above, in the photoelectric sensor provided in the pulse wave sensor unit 160, light can be cast onto the subject from a light emitting unit 311, and the light reflected by the subject can be received by a light receiving unit, and therefore biological information such as pulse wave signal can be detected.

Meanwhile, in the part excluding the detection window 2211, the light transmitting part 221 is covered by the light blocking part 222 from the subject side (bottom side on the face of FIG. 1). Thus, it is possible to limit the light incident on the pulse wave sensor unit 160. Therefore, the light that should be received, that is, the reflected light cast from the light emitting unit and reflected by the subject, can be received, while the reception of light which becomes a noise source, for example, ambient light such as sunlight and illumination light, can be restrained. Thus, the detection accuracy for biological information can be improved.

Also, the structure in which the light blocking part 222 covers the light transmitting part 221 can be grasped from a different perspective. Specifically, in the biological information measuring device 1 of the embodiment, in the state where the user (subject) is wearing the biological information measuring device 1, if the direction from the subject toward the case part 20 (in a narrow sense, the direction from the bottom case 22 toward the top case 21) is defined as a first direction DR1, the light transmitting part 221 is provided on the side of the first direction DR1 of the light blocking part 222, in the part excluding the detection window 2211.

Since the light transmitting part 221 transmits light, the possibility of inflow of light via the part where the light transmitting part 221 is provided must be considered for this part. Here, since the light transmitting part 221 is provided in the bottom case 22, the incident direction of the light to be considered is the direction from the subject toward the bottom case 22, that is, the first direction DR1. In this case, if the light transmitting part 221 is provided on the DR1 side of the light blocking part 222, the light to the light transmitting part 221 in the part excluding the detection window 2211 is considered to be affected by the light blocking by the light blocking part 222. Therefore, the incidence of light to be a noise source to the pulse wave sensor unit 160 can be restrained.

Also, as can be seen from the example of FIG. 1, the provision of the light transmitting part 221 on the DR1 side of the light blocking part 222 does not mean that the light transmitting part 221 is provided further to the DR1 side than the light blocking part 222 over the entire area of the light blocking part 222. For example, as in the area indicated by RB in FIG. 1, there may be an area where the light transmitting part 221 is not arranged on the DR1 side of the light blocking part 222. That is, the provision of the light transmitting part 221 on the DR1 side of the light blocking part 222 may mean that, when the light transmitting part 221 is provided, the light blocking part 222 is provided further to the side in the opposite direction of DR1, except the part of the detection window 2211. Specifically, in the area indicated by RA in FIG. 1, that is, in the area where the light transmitting part 221 is provided, excluding the detection window 2211, the light transmitting part 221 is further to the DR1 side than the light blocking part 222.

Here, the light transmitting part 221 is formed of a resin material, and the light blocking part 222 is formed of a glass-containing resin material which contains glass (in a narrow sense, glass fiber). Specifically, the light transmitting part 221 may be formed of polycarbonate, ABS resin or acrylic resin, and the light blocking part 222 may be formed of glass-containing polycarbonate, glass-containing ABS resin, or glass-containing acrylic resin.

That is, the light blocking part 222 according to the embodiment may be of FRP (fiber-reinforced plastics), and particularly GFRP (glass fiber-reinforced plastics) using glass fiber as fiber for reinforcement. The GFRP may use thermoplastic resin as a resin used together with glass fiber. In the embodiment, polycarbonate or ABS resin can be used as thermoplastic resin. Also, as the acrylic resin, thermoplastic acrylic resin and thermosetting acrylic resin are known, and both can be used in the embodiment. The GFRP is inexpensive among the FRPs and commonly available. Therefore, by employing the GFRP, it is possible to easily realize the light blocking part 222 according to the embodiment. Also, as the resin material of the GFRP, various resin materials can be used, such as polyester resin, vinyl ester resin, epoxy resin, and phenol resin. The light blocking part 222 according to the embodiment can use these broadly.

By employing the above-described structure, it is possible to realize a smaller size (thinner) and lighter weight, and specifically, to restrain the weight of the biological information measuring device 1 according to the embodiment to 60 g and form the biological information measuring device 1 with the planar size of the outer case (case part 20) being 6 cm or below and the case thickness being 15 mm or below. Here, the planar size of the case part 20 refers to the size in a plan view as viewed in the direction of observing from the subject side (user's wrist) in the state where the biological information measuring device 1 is worn by the user and thus used, as shown in FIG. 3(A), described later. The case thickness refers to the size in the direction orthogonal thereto (for example, in the DR1 direction in FIG. 1). Specifically, the maximum value of the length of the case part 20 in the plan view can be 6 cm or below, and the maximum value of the thickness in the DR1 direction can be 15 mm or below.

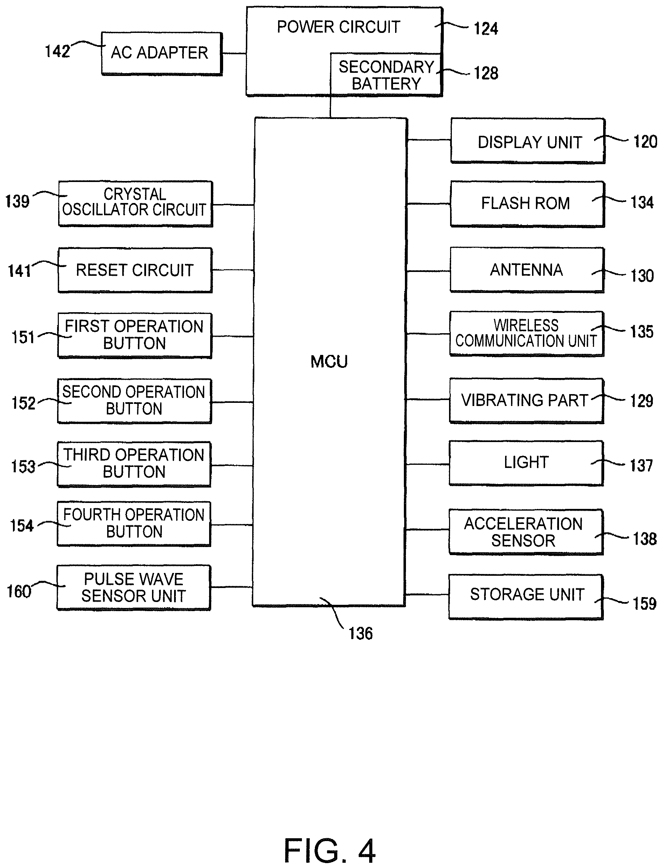

FIG. 4 is a block diagram showing an example of the system configuration of the biological information measuring device 1. As shown in FIG. 4, in the biological information measuring device 1, a power circuit 124, the display unit 120, a flash ROM 134, the antenna 130, a wireless communication unit 135, the vibrating part 129, a light 137, an acceleration sensor 138, a crystal oscillator circuit 139, a reset circuit 141, a storage unit 159, the pulse wave sensor unit 160, and operation buttons 151 to 154 are connected to the MCU 136.

The MCU 136 has a memory for storing a program inside and is configured to control each part of the biological information measuring device 1 and also perform processing to generate time information, tap detection processing as described later, processing to store the traveling state of the user, and speed calculation processing or the like. The power circuit 124 recharges the secondary battery 128 when connected to an AC adapter 142 via a connection terminal (not shown). The secondary battery 128 supplies drive power to the display unit 120, the antenna 130 and the like.

In the flash ROM 134, time difference information is stored, for example. The time difference information is information which defines time difference data (amount of correction to UTC associated with coordinate values (for example, latitude and longitude) or the like). Also, as described below, the correlation between body vibration frequency and speed is recorded in the flash ROM 134 as well.

The antenna 130 performs processing to acquire satellite information such as satellite trajectory information, GPS time information, or location information or the like included in a navigation message from a satellite signal in a 1. 5-GHz band, for example.

The wireless communication unit 135 performs wireless communication between the biological information measuring device 1 and a personal computer or the like and thus can transmit log data stored in the biological information measuring device 1 to the personal computer or the like. The light 137 is used to facilitate visual recognition by the user at night or the like, by casing light to the display unit 120 in response to an operation on an operation button by the user. Although not illustrated, a buzzer used to report the completion of setting processing by the user is provided as well.

The crystal oscillator circuit 139 is a crystal oscillator circuit with a temperature compensation circuit and generates a reference clock signal of a substantially constant frequency regardless of temperature. The reset circuit 141 is used to reset the measuring state of the biological information measuring device 1 in response to a predetermined operation by the user.

Here, the operation modes of the biological information measuring device 1 according to the one embodiment and the main functions provided for the operations buttons 151 to 154 with respect to switching among the operation modes will be described in detail.

The biological information measuring device 1 according to the embodiment has at least three operation modes, that is, a "time display mode" as a first mode, a "measuring mode" as a second mode, and a "heart rate display mode" as a third mode.

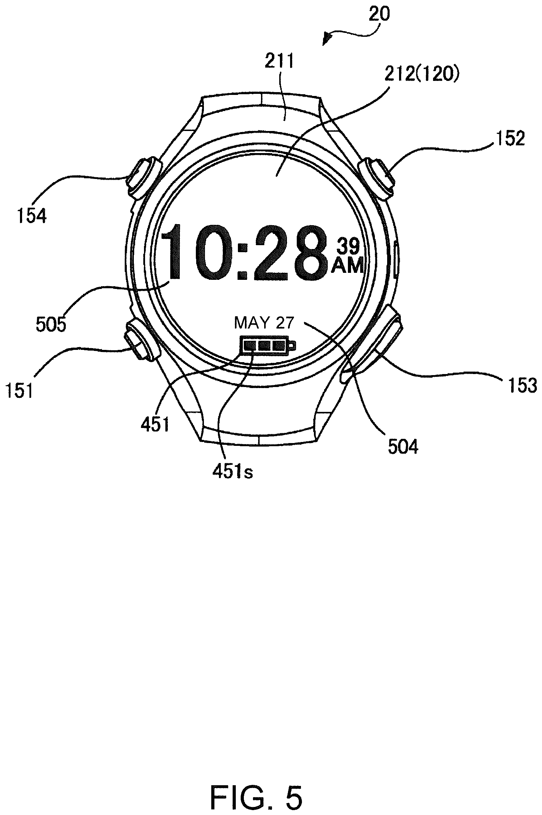

FIG. 5 is a view showing a display example on the display unit 120 when the biological information measuring device 1 is in the "time display mode". The time display mode is the operation mode that is set when using the biological information measuring device 1 mainly for displaying time (as a wristwatch).

In the time display mode, the pulse wave sensor unit 160 (photoelectric sensor) and the antenna 130 are set in a halt state by the MCU 136, and the processing involved in the acquisition of a pulse wave signal and the acquisition of satellite information is not executed. Then, time information 505 is displayed on the display unit 120 as a main display, as shown in FIG. 5. In the example of FIG. 5, date information 504 is displayed on the display unit 120 as well.

A battery display 451 shown in FIG. 5 is the display showing the remaining capacity of the secondary battery 128. A fewer number of rectangular marks 451s displayed indicates a less remaining capacity of the secondary battery 128.

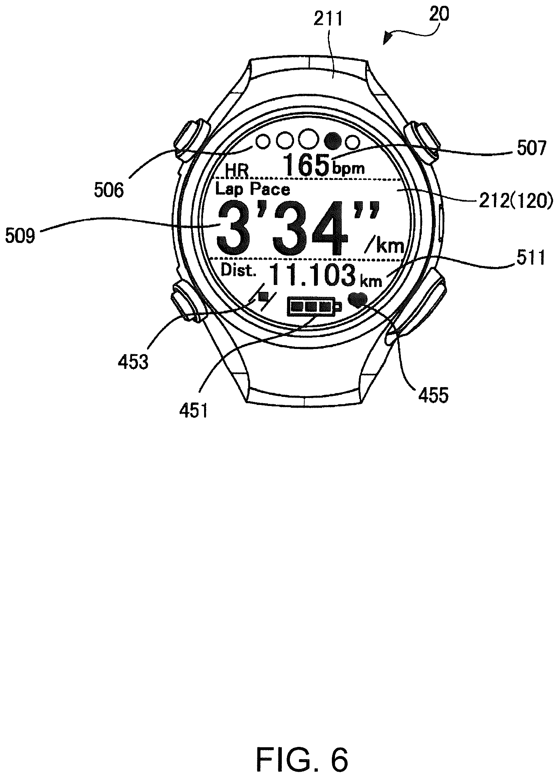

FIG. 6 is a view showing a display example on the display unit 120 when the biological information measuring device 1 is in the "measuring mode". The measuring mode is the operation mode that is set when using the biological information measuring device 1 as a so-called running watch.

In the measuring mode, the pulse wave sensor unit 160 and the antenna 130 are set in an operating state by the MCU 136, and the processing involved in the acquisition of a pulse wave signal and the acquisition of satellite information is executed. A satellite display 453 shown in FIG. 6 is the display indicating that the antenna 130 is in the operating state. A heart display 455 shown in FIG. 6 is the display indicating the pulse wave sensor unit 160 is in the operating state.

Also, a zone display 506 is the display indicating which of a plurality of (in this example, five) preset heartbeat zones (numerical range prescribed by an upper limit value and lower limit value; heartbeat range) the current heart rate of the user belongs to.

Here, the five circular displays forming the zone display 506 correspond in order from the left to a "heartbeat zone 1", "heartbeat zone 2", "heartbeat zone 3", "heartbeat zone 4", and "heartbeat zone 5". The circular display that is lit (displayed in black in the illustration) is the heartbeat zone to which the current heart rate of the user belongs. In the example shown in FIG. 6, the heart rate of the user belongs to the heartbeat zone 4. Hereinafter, a heartbeat zone is simply referred to as a "zone".

In the measuring mode, the MCU 136 calculates the number of times the heart beats (hereinafter referred to as "heart rate") during a predetermined time (in this example, one minute) on the basis of a pulse wave signal acquired by the pulse wave sensor unit 160, generate heart rate information indicating this heart rate, stores the heart rate information in the storage unit 159, and displays heart rate information 507 on the display unit 120 as shown in FIG. 6.

The heart rate may be an actually measured value or may be an estimate value. Also, the time interval at which the heart rate information is calculated and stored, and the update interval of the heart rate information 507 displayed on the display unit 120 may be arbitrary. The "HR" shown in FIG. 6 is the abbreviation of heart rate, and "bpm" is the abbreviation of "beats per minute".

Also, in the measuring mode, the MCU 136 calculates lap pace information indicating the time required for running 1 km (hereinafter referred to as "lap pace"), for example, on the basis of the satellite information and time information, and displays lap pace information 509 on the display unit 120 as shown in FIG. 6. The example shown in FIG. 6 shows that the lap pace if 3 minutes 34 seconds [/km].

Moreover, in the measuring mode, the MCU 136 calculates cumulative moving distance information indicating the cumulative moving distance, for example, on the basis of the satellite information, and displays cumulative moving distance information 511 on the display unit 120 as shown in Fig. 6. The example shown in FIG. 6 shows that the cumulative moving distance is 11.103 km. In FIG. 6, "Dist." is the abbreviation of distance.

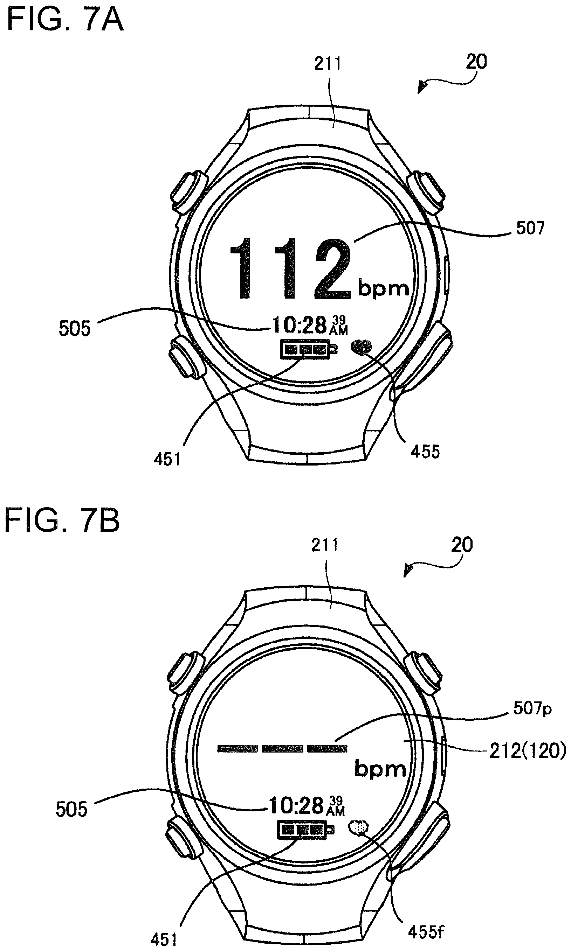

FIG. 7(A) is a view showing a display example on the display unit 120 when the biological information measuring device 1 is in the "heartbeat display mode". The heartbeat display mode is the operation mode that is set when the user temporarily wants to know the heart rate at the time point.

The main difference between the heartbeat display mode and the measuring mode is that, in the heartbeat display mode, the antenna 130 is set in the halt state and the heart rate information is not stored in the storage unit 159. Thus, according to the heartbeat display mode, the power consumption and processing load required for the processing to set the antenna 130 in the operating mode, acquire satellite information and calculate various kinds of information can be saved, and excessive use of the storage capacity of the storage unit 159 is prevented.

In the heartbeat display mode, since the pulse wave sensor unit 160 is set in the operating state, the heart display 455 is displayed on the display unit 120 as shown in FIG. 7 (A), as in the measuring mode.

Then, in the heartbeat display mode, the MCU 136 calculates the heart rate on the basis of a pulse wave signal acquired by the pulse wave sensor unit 160, and displays the heart rate information 507 indicating this heart rate on the display unit 120 as a main display, as shown in FIG. 7(A).

As in the case of the measuring mode, the heart rate may be an estimate value or may be an actually measured value. Also, as in the example shown in FIG. 7 (A), the time information 505 may be displayed with the heart rate information 507 on the display unit 120.

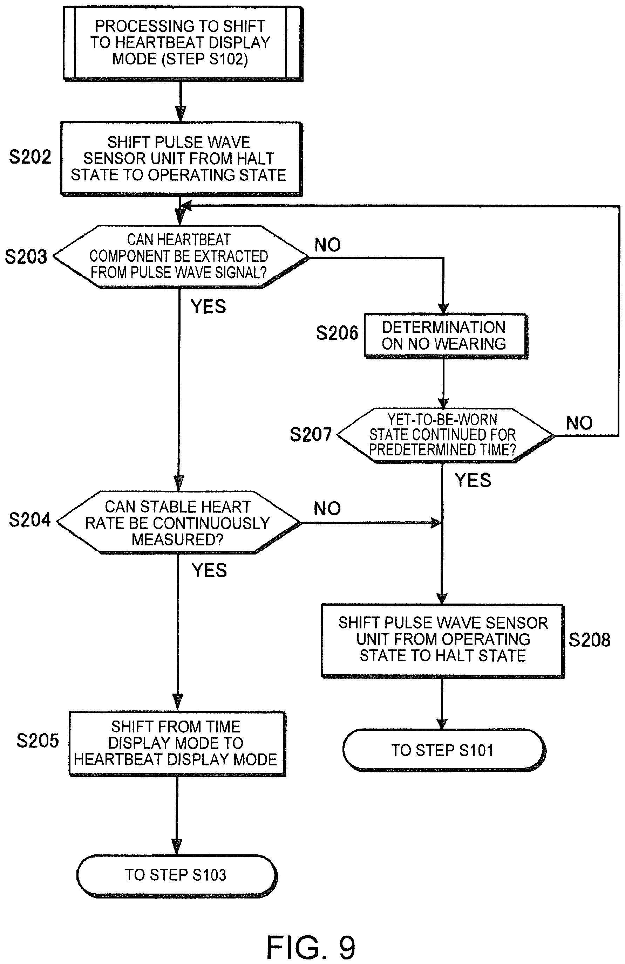

As will be described in detail later, when shifting from the time display mode to the heartbeat display mode in response to a predetermined operation, the biological information measuring device 1 needs a predetermined preparation time after the pulse wave sensor unit 160 is set in the operating state, in order to actually calculate and display the heart rate information from the pulse wave signal. The processing executed by the MCU 136 during this preparation time will be described later with reference to FIG. 9.

During this preparation time, a "preparation screen" shown in FIG. 7(B) is displayed on the display unit 120, for example. On the preparation screen, heartbeat preparation information 507p "---" is displayed at the position where the heart rate information 507 should be displayed in the heartbeat display mode. Also, a flashing heart display 455f is displayed at the position where the heart display 455 should be displayed in the heartbeat display mode.

Incidentally, the biological information measuring device 1 is usually set in the time display mode shown in FIG. 5 and shifts to other modes, described later, in response to an operation on the operation buttons 151 to 154. Of the functions which the operation buttons 151 to 154 have, mainly the function related to shifting to the operating mode will be described below.

The operation buttons 151 to 154 for the user to manually operate are provided, protruding outward on the lateral sides of the case part 20, for example, as shown in FIG. 5. Specifically, the first operation button (hereinafter referred to as the "first button") 151 and the fourth operation button 154 (hereinafter referred to as the "fourth button") are arranged within the range from the position of 6 o'clock to the position of 12 o'clock including the position of 9 o' clock, and the second operation button (hereinafter referred to as the "second button") 152 and the third operation button (hereinafter referred to as the "third button") 153 are arranged within the range from the position of 12 o'clock to the position of 6 o'clock including the position of 3 o'clock.

One of the functions which the first button 151 has is the function of shifting the device from the "time display mode" to the "heartbeat display mode". Specifically, when the biological information measuring device 1 is in the time display mode, if an operation signal indicating that a short press operation (hereinafter simply referred to as "short press") is performed on the first button 151 is outputted from the first button 151, the MCU 136 executes the processing to shift the biological information measuring device 1 to the heartbeat display mode.

One of the functions which the second button 152 has is the function of shifting the device from the "time display mode" to the "measuring mode". Specifically, when the biological information measuring device 1 is in the time display mode, if an operation signal indicating that a long press operation (hereinafter simply referred to as "long press") is performed on the second button 152 is outputted from the second button 152, the MCU 136 executes the processing to shift the biological information measuring device 1 to the measuring mode. Also, a configuration in which the shift to the measuring mode is carried out by a short press operation instead of the long press operation on the second button 152 may be employed.

One of the functions which the third button 153 has is the function of resetting the measuring state in the measuring mode. Specifically, when the biological information measuring device 1 is in the measuring mode, if an operation signal indicating that the third button 153 is long-pressed is outputted from the third button 153, the MCU 136 executes the processing to reset the measuring state by the reset circuit 141.

The measuring state reset by the reset circuit 141 is the measuring state in which lap pace information, cumulative moving distance information and the like are initialized, and equivalent to the state where the user does not move at all after a shift to the measuring mode. This measuring state is referred to as a measuring initial state.

In the biological information measuring device 1 according to the embodiment, the fourth button 154 does not have any function related to the switching among operation modes. The fourth button 154 has the function of setting switching on/off the light 137. Specifically, if an operation signal indicating that the fourth button 154 is short-pressed is outputted from the fourth button 154, the MCU 136 executes the processing to cause the light 137 to emit light and cast the light on the display unit 120 for a predetermined time.

The operation buttons 151 to 154 are used to switch among operation modes (measuring mode, time display mode and the like) and to carry out display settings on the display unit 120 and various setting inputs.

The description goes back to FIG. 4.

The vibrating part 129 is a device for notifying the user of a warning or the like via vibrations. For example, the vibrating part 129 has an eccentric rotating weight part, and when a current flows through the vibrating part 129, this rotating weight part rotates to generate vibrations. As the vibrations are transmitted to the user's arm via the biological information measuring device 1, the user can be notified.

The acceleration sensor 138 is a sensor provided on the circuit board 40 and capable detecting acceleration in three axial directions. That is, as the three axial direction, specifically, in the state where the user wears the biological information measuring device 1 on an arm and runs with the thumb facing up, the traveling direction of the user is an X-axis direction, the up/down movement (gravitational) direction of the user is a Y-axis direction, and the left/right movement direction of the user is a Z-axis direction.

The pulse wave sensor unit 160, having the photoelectric sensors for measuring a pulse wave as biological information, as described above, acquires a pulse wave signal of the user and outputs the pulse wave signal to the MCU 136.

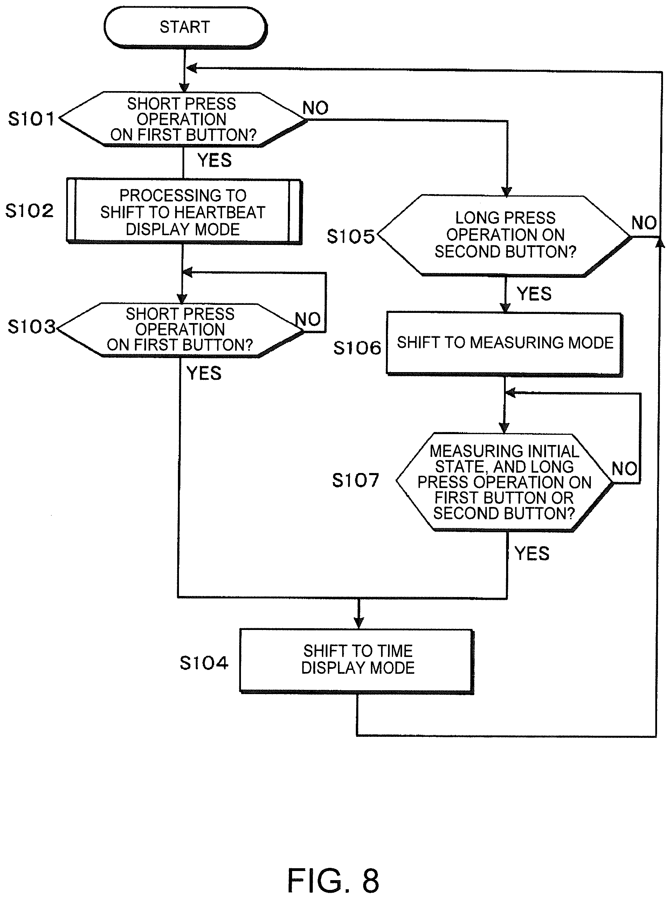

FIG. 8 is a view showing a flowchart of operation mode switching processing. The biological information measuring device 1 according to the one embodiment is usually set in the time display mode shown in FIG. 5 as described above, and shifts to other modes in response to an operation on the operation buttons 151 to 154. Hereinafter, an example of the operation mod switching processing will be specifically described with reference to FIG. 8.

In the state where the biological information measuring device 1 is set in the time display mode, the MCU 136 determines whether an operation signal corresponding to a short press operation on the first button 151 is inputted or not (Step S101). If the result of the determination in Step S101 is positive, in other words, if an operation corresponding to a short press operation on the first button 151 is inputted, the MCU 136 executes the "processing to shift the biological information measuring device 1 to the heartbeat display mode" (Step S102).

FIG. 9 is a view showing the subroutine of Step S102 (flowchart of processing to shift to the heartbeat display mode).

The processing shown in FIG. 9 is the processing in view of the following problem. That is, if the first button 151 is short-pressed due to a certain factor in the state where the biological information measuring device 1 is not worn by the user, the pulse wave sensor unit 160 is set in the operating state despite no acquisition of the pulse wave signal of the user and therefore power consumption and processing load increase unnecessarily. Such a phenomenon can adversely affect the power consumption and processing load reduction effect achieved by the heartbeat display mode, which is one of the characteristics of the biological information measuring device 1 according to the embodiment.

Thus, in the biological information measuring device 1 according to the embodiment, the processing of the flowchart shown in FIG. 9 is executed, thus determining whether the biological information measuring device 1 is worn by the user or not. If the biological information measuring device 1 is yet to be worn, no shift to the heartbeat display mode is made and the time display mode is maintained.

The processing of the flowchart shown in FIG. 9 is the processing executed by the MCU 136 during the preparation time described above with reference to FIG. 7(B).

First, the MCU 136 sets the pulse wave sensor unit 160 in the operating state (Step S202). Subsequently, the MCU 136 determines whether a heartbeat component can be extracted from the pulse wave signal outputted from the pulse wave sensor unit 160 or not (Step S203). If the result of the determination in Step S203 is negative, in other words, if a heartbeat component cannot be extracted, the MCU 136 determines that the biological information measuring device 1 is not worn by the user (yet to be worn) (Step S206). Here, the MCU 136 determines whether the yet-to-be-worn state has continued for a predetermined time or not (Step S207). If the result of the determination in Step S207 is negative, in other words, if the yet-to-be-worn state has not continued for a predetermined time, the MCU 136 returns to the processing of Step S203. On the other hand, if the result of the determination in Step S207 is positive, in other words, if the yet-to-be-worn has continued for a predetermined, the MCU 136 sets the pulse wave sensor unit 160 in the halt state (Step S208), shifts the device to the display screen of the time display mode without shifting to the heartbeat display mode, and returns to the processing of Step S101.

Meanwhile, if the result of the determination in Step S203 is positive, in other words, if a heartbeat component can be extracted, whether a stable heart rate can be continuously measured (a sudden irregularity in the pulse wave signal is absent) or not is determined (Step S204). If the result of the determination in Step S204 is negative, in other words, if a stable heart rate cannot be continuously measured (there is a sudden irregularity in the pulse wave signal), the processing shifts to Step S208. On the other hand, if the result of the determination in Step S204 is positive, in other words, if a stable heart rate can be continuously measured (a sudden irregularity in the pulse wave signal is absent), the biological information measuring device 1 is set in the heartbeat display mode (Step S205) and the processing shifts to Step S103, described later.

After the processing of Step S205 is completed, in other words, after the biological information measuring device 1 is shifted to the heartbeat display mode, the MCU 136 determines again whether an operation signal corresponding to a short press operation on the first button 151 is inputted or not (Step S103). If the result of the determination in Step S103 is positive, in other words, if an operation signal corresponding to a short press operation on the first button 151 is inputted, the MCU 136 executes the processing to shift the biological information measuring device 1 to the time display mode (Step S104) and returns to the processing of Step S101.

On the other hand, if the result of the determination in Step S103 is negative, in other words, if an operation signal corresponding to a short press operation on the first button 151 is not inputted, the MCU 136 returns to the processing of Step S103. That is, the biological information measuring device 1 maintains the heartbeat display mode.

Meanwhile, if the result of the determination in Step S101 is negative, in other words, if an operation signal corresponding to a short press operation on the first button 151 is not inputted, the MCU 136 determines whether an operation signal corresponding to a long press operation on the second button 152 is inputted or not (Step S105). If the result of the determination in Step S105 is negative, in other words, if an operation signal corresponding to a long press operation on the second button 152 is not inputted, the MCU 136 returns to the processing of Step S101.

On other hand, if the result of the determination in Step S105 is positive, in other words, if an operation signal corresponding to a long press operation on the second button 152 is inputted, the MCU 136 executes the processing to shift the biological information measuring device 1 to the measuring mode (Step S106).

After the processing of Step S106 is completed and the biological information measuring device 1 is shifted to the measuring mode, the MCU 136 determines whether the biological information measuring device 1 is in the above "measuring initial state" and an operation signal corresponding to a long press operation on the first button 151 or the second button 152 is inputted or not (Step S107). If the result of the determination in Step S107 is positive, in other words, if an operation signal corresponding to a short press operation on the first button 151 is inputted, the MCU 136 executes the processing to shift the biological information measuring device 1 to the time display mode (Step S104) and returns to the processing of Step S101.

On the other hand, if the result of the determination in Step S107 is negative, in other words, if the biological information measuring device 1 is not in the measuring initial state, or even if it is in the measuring initial state, if an operation signal corresponding to a long press operation on the first button 151 or the second button 152 is not inputted, the processing returns to Step S107. That is, the biological information measuring device 1 maintains the measuring mode.

After the processing of Step S104 is completed and the biological information measuring device 1 is shifted to the time display mode, the processing returns to Step S101.

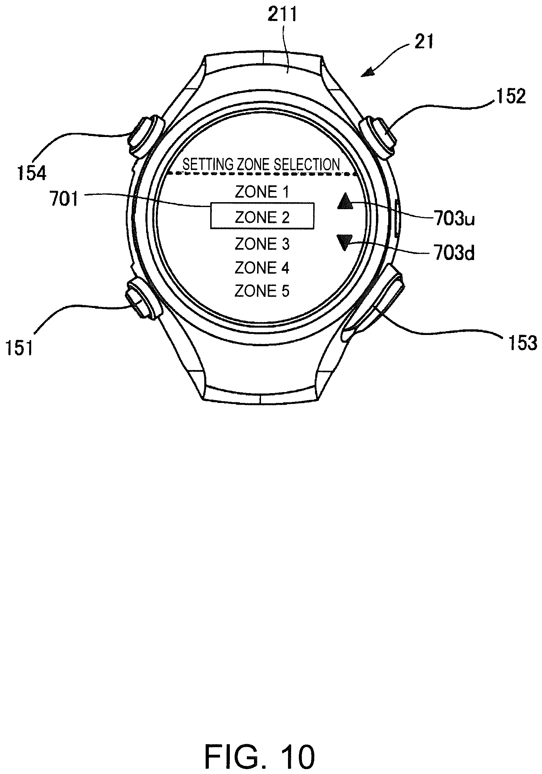

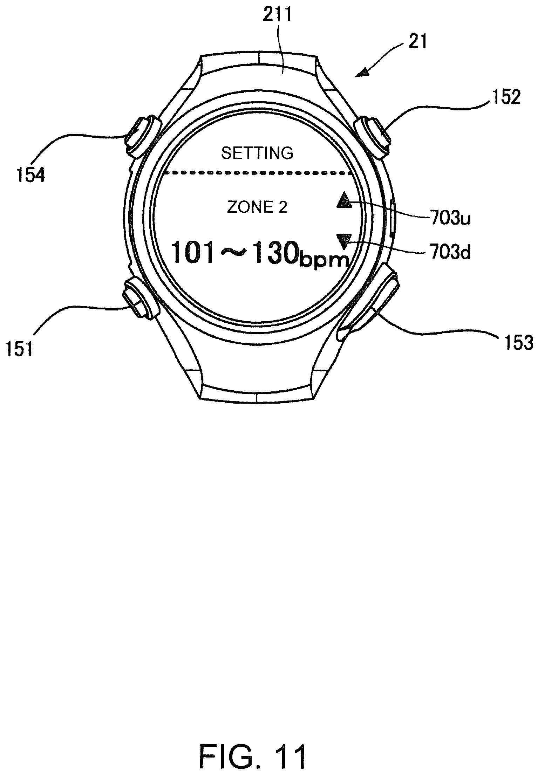

Incidentally, the above "zone" can be specifically set by the user as follows. FIG. 10 and FIG. 11 are views showing an example of the screen displayed on the display unit 120 when the user sets a zone.

If a predetermined operation is carried out using the operation buttons 151 to 154 when the biological information measuring device 1 is in the time display mode, a "setting zone selection screen" shown in FIG. 10 is displayed on the display unit 120. The user selects and decides a zone of a desired numerical range setting, by moving up and down a selection mark 701 on the setting zone selection screen. An up mark 703u indicating an up movement of the selection mark 701 corresponds to the second button 152, and a down mark 703d indicating a down movement of the selection mark 701 corresponds to the third button 153. Also, in order to decide the zone to which the selection mark 701 is applied, as a setting target, the first button 151 is used, for example.

If, for example, the zone 2 is selected and decided as a setting target on the setting zone selection screen shown in FIG. 10, a numerical range setting screen shown in FIG. 11 is displayed, for example, on the display unit 120. The user sets the numerical range of this zone (in this example, the zone 2) by a predetermined operation using the operation buttons 151 to 154.

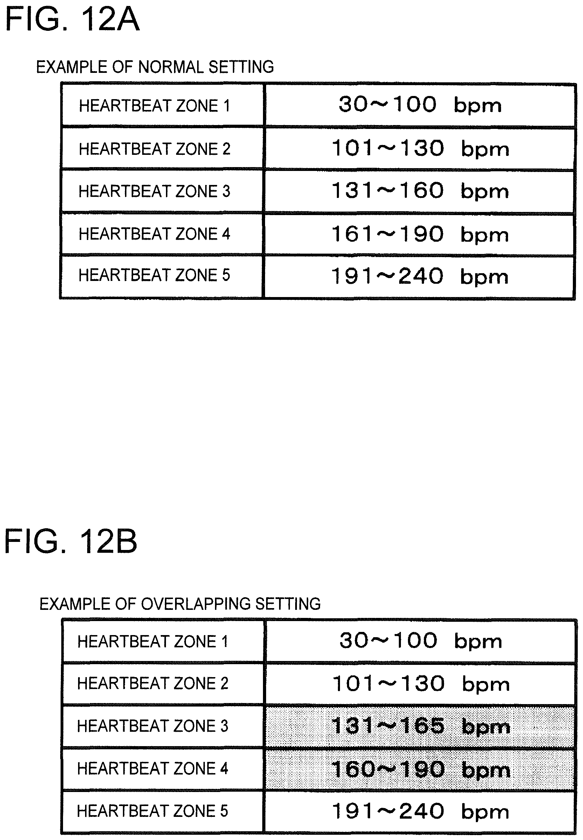

FIG. 12 is a view for explaining a setting example of the numerical ranges of zones.

The setting example (example of normal setting) shown in FIG. 12(A) is an example in which the numerical ranges of the respective zones are set in such a way as not to overlap with each other (exclusively). That is, in the example shown in FIG. 12(A), the zone 1 is set to the numerical range of 30 to 100 bpm, the zone 2 to 101 to 130 bpm, the zone 3 to 131 to 160 bpm, the zone 4 to 161 to 190 bpm, and the zone 5 to 191 to 240 bpm.

The setting example (example of overlapping setting) shown in FIG. 12 (B) is an example in which the numerical ranges of at least two or more zones (in this example, two zones) are set in an overlapping manner. That is, in the example shown in FIG. 12(B), the zone 1 is set to the numerical range of 30 to 100 bpm, the zone 2 to 101 to 130 bpm, the zone 3 to 131 to 165 bpm, the zone 4 to 160 to 190 bpm, and the zone 5 to 191 to 240 bpm. In FIG. 12 (B), the shaded zones are zones set to numerical ranges overlapping each other.

Here, the zone display corresponding to the example normal setting shown in FIG. 12 (A) is the zone display 506 shown in FIG. 6. That is, if the heart rate at the time point is 165 bpm in the measuring mode, the fourth circular display from the left indicating the zone 4 to which this heart rate of 165 belongs is lit (displayed in black in the illustration). By visually recognizing the zone display 506, the user can easily grasp which of the zones his/her heart rate belongs to, each zone being set to a desired numerical range by the user.

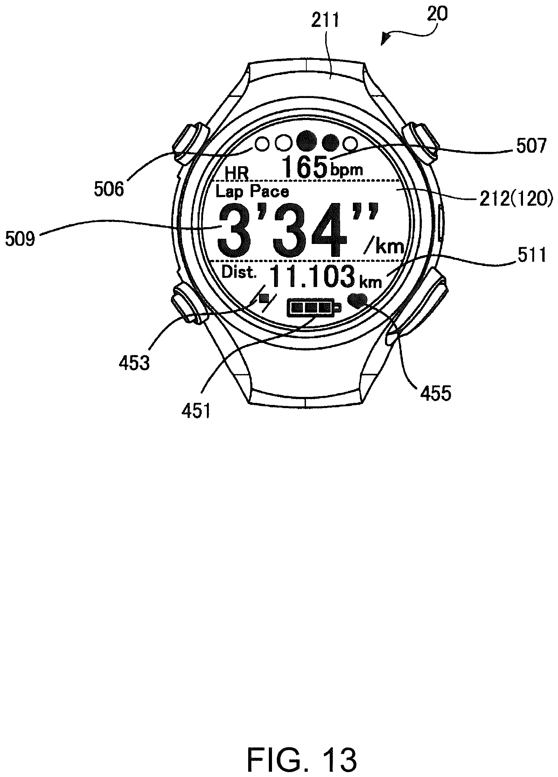

On the other hand, the zone display corresponding to the example of overlapping setting shown in FIG. 12 (B) is the zone display 506 shown in FIG. 13. That is, FIG. 13 is a view showing a display example on the display unit 120 in the measuring mode in the case where the numerical ranges of zones are set in an overlapping manner. As shown in FIG. 13, if the heart rate at the time point is 165 bpm in the measuring mode, this heart rate of 165 belongs to the zone 3 and the zone 4. Therefore, the third circular display from the left indicating the zone 3 and the fourth circular display from the left indicating the zone 4 are lit (displayed in black in the illustration). By visually recognizing the zone display 506, the user can easily grasp which of the zones his/her heart rate belongs to, each zone being set to a desired numerical range by the user.

As the numerical ranges of zones are thus set in an overlapping manner, for example, flexible running becomes easier in which the zone of the highest heart rate is achieved in a specific section in the course of running, whereas heart rates in the upper half of the numerical range of the heart rate zone that is immediately below are allowed in other sections.

In the traditional configuration of the biological information measuring device, such zone setting cannot be achieved. Moreover, in the traditional configuration of the biological information measuring device, since each of a plurality of zones cannot be expressed individually as in the zone display 506, only the zones set to numerical ranges exclusively with respect to each other can be dealt with.

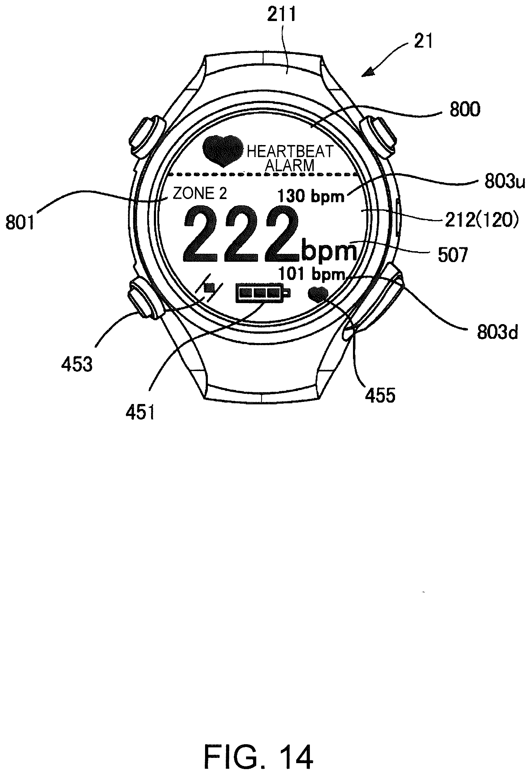

FIG. 14 is a view showing a display example on the display unit 120 when a "heartbeat alarm" giving a warning to the user is actuated. The "heartbeat alarm" is the function of displaying a warning screen as shown in FIG. 14 on the display unit 120 by the MCU 136 when the heart rate of the user belongs to a "warning zone" which is one or a plurality of zones to be warned of. When the heartbeat alarm is actuated, the display unit 120 functions as a warning unit for giving a warning to the user. Here, the "warning zone" can be set by the user carrying out a predetermined operation using the operation buttons 151 to 154.

Also, when the heartbeat alarm is actuated, a predetermined warning sound may be outputted from a speaker (not illustrated) to draw the user' s attention, or vibrations may be transmitted to the user' s arm by the vibrating part 129 to draw the user's attention.

In the example of the warning screen shown in FIG. 14, a warning display 800 indicating that the heartbeat alarm is actuated, the heart rate information 507 at the time point, a warning zone display 801 indicating the warning zone, and a lower limit value 803d and an upper limit value 803u of the warning zone are displayed on the display unit 120.

The above example is an example in which the heartbeat alarm is actuated when the heart rate of the user belongs to the warning zone. However, a configuration in which the heartbeat alarm is actuated when the heart rate of the user goes out of a predetermined zone (target zone) may also be employed. In this case, the user sets one or a plurality of target zones excluded from zones to be warned of, and when the heart rate does not belong to the target zone, the MCU 136 controls the warning unit (display unit 120, vibrating part 129 and the like) to give a warning. The heartbeat alarm in which the warning zone is set and the heartbeat alarm in which the target zone is set are substantially equivalent to each other.

As described above, according to one embodiment of the invention, a biological information measuring device in which the numerical range of heart rate corresponding to each zone can be set in an overlapping manner between a plurality of zones and in which the zones set in this manner can be properly presented to the user, can be provided.

While the embodiment is described in detail as above, a person skilled in the art can readily understand that a number of modifications can be made without substantially departing from the new matters and advantage effects of the invention. Therefore, all such modifications are considered to be included in the scope of the invention. For example, a term described along with a different term with a broader or same meaning at least once in the specification or drawings can be replaced with the different term at any point in the specification or drawings. Also, the configurations and operations of the biological information measuring device are not limited to those described in the embodiment and can be implemented with various modifications.

### [First Modification]

In the biological information measuring device 1 according to the one embodiment described above, the processing contents of Step S103 in the flowchart shown in FIG. 8 may be changed as follows.

That is, the condition for proceeding from Step S103 to the processing of Step S104 and shift to the time display mode may be "the lapse of a predetermined time such as one minute, for example" instead of "a short press operation on the first button 151".

In this case, the MCU 136 determines in Step S103 whether a predetermined time has passed or not. If the result of the determination is positive, the MCU 136 proceeds to the processing of Step S104. Also, a configuration in which the MCU 136 proceeds to the processing of Step S104 with the lapse of a predetermined time in this manner, whereas before the lapse of the predetermined time, the MCU 136 proceeds to the processing of Step S104 in response to a short press operation on the first button 151, may be employed.

### [Second Modification]

In the biological information measuring device 1 according to the one embodiment described above, the processing contents of Step S107 in the flowchart shown in FIG. 8 may be changed as follows.

That is, the condition for proceeding from Step S107 to the processing of Step S104 and shift to the time display mode may be "the lapse of a predetermined time such as one hour, for example" instead of "being in the measuring initial state and a long press operation on the first button 151 or the second button 152".

In this case, the MCU 136 determines in Step S107 whether a predetermined time has passed or not. If the result of the determination is positive, the MCU 136 proceeds to the processing of Step S104. Also, a configuration in which the MCU 136 proceeds to the processing of Step S104 with the lapse of a predetermined time in this manner, whereas before the lapse of the predetermined time, the MCU 136 proceeds to the processing of Step S104 in response to a long press operation on the first button 151 or the second button 152 in the measuring initial state, may be employed.

### [Application Examples]

In addition to the above modes, a fourth mode in which each of the antenna 130 and the pulse wave sensor unit 160 can be individually set in the operating state/halt state may be added.

The drive time also changes depending on the operating circumstances. For example, if positioning every second by the GPS (antenna 130) and measurement of pulse wave information by the pulse wave sensor unit 160 are carried out simultaneously, the biological information measuring device 1 can be driven for 20 hours. Also, if the pulse wave sensor unit 160 is in the halt state and the antenna 130 is in the operating state to perform GPS positioning every second, the biological information detection device 1 can be driven for 24 hours. Moreover, if the antenna 130 is in the halt state and measuring by the pulse wave sensor unit 160 is carried out, the biological information detection device 1 can be driven for 60 hours.

### Reference Signs List

1 ... biological information measuring device, 30 ...antenna, 40 ... circuit board, 42 ... panel frame, 44 ... circuit case, 120 ... display unit, 124 ... power circuit, 128 ... secondary battery, 129 ... vibrating part, 130 ... antenna, 135 ... wireless communication unit, 137 ... light, 138 ... acceleration sensor, 139 ... crystal oscillator circuit, 141 ... reset circuit, 142 ... AC adapter, 151 ... first operation button, 152 ... second operation button, 153 ... third operation button, 154 ... fourth operation button, 159 ... storage unit, 160 ... pulse wave sensor unit, 211 ... trunk part, 212 ... glass plate, 221 ... light transmitting part, 2211 ... detection window, 222 ... light blocking part, 311 ... light emitting unit, 451 ... battery display, 453 ... satellite display, 455, 455f ... heart display, 504 ... date information, 505 ... time information, 506 ... zone display, 507 ... heart rate information, 507p ... heartbeat preparation information, 509 ... lap pace information, 511 ... cumulative moving distance information

## Claims

1. A biological information measuring device comprising:
a pulse wave sensor unit which detects a pulse wave of a living body and outputs a pulse wave signal;
a heart rate information generation unit which generates heart rate information indicating a heart rate on the basis of the pulse wave signal;
a time information generation unit which generates time information indicating time;
a storage unit capable of storing the heart rate information;
an operation unit which outputs an operation signal corresponding to an input operation by a person;
a display unit; and
a control unit which displays the time information on the display unit in a first mode, writes the heart rate information in the storage unit in a second mode, and displays the heart rate information on the display unit without writing the heart rate information in the storage unit in a third mode,
wherein the control unit starts processing to shift the biological information measuring device from the first mode to the third mode if the operation signal indicates a predetermined operation.

2. The biological information measuring device according to claim 1, wherein the control unit sets the pulse wave sensor unit in a halt state in the first mode, and sets the pulse wave sensor unit in an operating state in the second mode and the third mode.

3. The biological information measuring device according to claim 1 or 2, comprising a receiving unit which receives position information from a satellite, wherein the control unit sets the receiving unit in an operating state in the second mode and sets the receiving unit in a halt state in the third mode.

4. The biological information measuring device according to one of claims 1 to 3, wherein the operation unit has a plurality of buttons including a first button, and the predetermined operation is an operation on the first button.

5. The biological information measuring device according to claim 4, wherein the plurality of buttons includes a second button, and
the control unit shifts the biological information measuring device from the first mode to the second mode if the operation signal indicates an operation on the second button.

6. The biological information measuring device according to one of claims 1 to 5, wherein the control unit shifts the biological information measuring device from the third mode to the first mode when a predetermined time has passed after the shift to the third mode.

7. The biological information measuring device according to claim 6, wherein the control unit sets the predetermined time on the basis of the operation signal.

8. The biological information measuring device according to one of claims 4 to 7, wherein the control unit shifts the biological information measuring device from the third mode to the first mode if the operation signal indicates an operation on the first button after the shift to the third mode.

9. The biological information measuring device according to one of claims 1 to 8, comprising a determination unit which determines whether the biological information measuring device is worn on the living body or not, on the basis of the pulse wave signal,
wherein the control unit maintains the first mode if it is determined by the determination unit that the biological information measuring device is not worn on the living body, even when processing to shift from the first mode to the third mode is started.
